# EUROPEAN PATENT SPECIFICATION

(11) **EP 1 880 023 B1**
(45) Date of publication and mention of the grant of the patent: **27.07.2011**
(21) Application number: 06757697.5
(22) Date of filing: 11.05.2006
(51) Int. Cl.: C12Q 1/68

(54) **METHOD FOR QUANTITATIVE ANALYSIS OF METHYL CYTOSINE IN DNA AND USES THEREOF**
VERFAHREN ZUR QUANTITATIVEN ANALYSE VON METHYLCYTOSIN IN DNA UND VERWENDUNGEN DAVON
PROCÉDÉ POUR L'ANALYSE QUANTITATIVE DE MÉTHYLCYTOSINE DANS DE L'ADN ET UTILISATIONS ASSOCIÉES

(30) Priority: 11.05.2005 KR 20050039210
(43) Date of publication of application: 23.01.2008
(73) Proprietor: Korea Research Institute of Standards and Science, Daejeon-city 305-340 (KR)
(72) Inventor: YANG, In-Chul, Yuseong-gu, Daejeon 305-756 (KR); JANG, Sung-Moon, Seoul 150-072 (KR); SHI, Lian-Hua, Yuseong-gu Daejeon 305-340 (KR); PARK, Sang-Ryoul, Yuseong-gu, Daejeon 305-340 (KR)
(74) Representative: Hiebl, Inge Elisabeth
(86) International application number: PCT/KR2006/001765
(87) International publication number: WO 2006/121295

(56) References cited:
- US-B1- 6 331 393
- FRAGA MARIO F ET AL: "High-performance capillary electrophoretic method for the quantification of 5-methyl 2'-deoxycytidine in genomic DNA: application to plant, animal and human cancer tissues." ELECTROPHORESIS JUN 2002, vol. 23, no. 11, June 2002 (2002-06), pages 1677-1681, XP002483854 ISSN: 0173-0835
- FRANK LYKO: "Novel methods for analysis of genomic DNA methylation" ANALYTICAL AND BIOANALYTICAL CHEMISTRY, SPRINGER-VERLAG, BE, vol. 381, no. 1, 1 January 2005 (2005-01-01), pages 67-68, XP019327069 ISSN: 1618-2650
- FRAGA M F ET AL: "DNA METHYLATION: A PROFILE OF METHODS AND APPLICATIONS" BIOTECHNIQUES, INFORMA LIFE SCIENCES PUBLISHING, WESTBOROUGH, MA, US, vol. 33, no. 3, 1 September 2002 (2002-09-01), pages 632,634,636-649, XP001107174 ISSN: 0736-6205
- CHRISTINA DAHL ET AL: "DNA methylation analysis techniques" BIOGERONTOLOGY, KLUWER ACADEMIC PUBLISHERS, DO, vol. 4, no. 4, 1 August 2003 (2003-08-01), pages 233-250, XP019230475 ISSN: 1573-6768
- VOSS KARL ET AL: "Use of capillary electrophoresis to study methylation patterns in DNA" PROC SPIE INT SOC OPT ENG; PROCEEDINGS OF SPIE - THE INTERNATIONAL SOCIETY FOR OPTICAL ENGINEERING 1996 SOCIETY OF PHOTO-OPTICAL INSTRUMENTATION ENGINEERS, BELLINGHAM, WA, USA, vol. 2680, 1996, pages 214-218, XP002483855
- BOYD VICTORIA L ET AL: "Methylation-dependent fragment separation: Direct detection of DNA methylation by capillary electrophoresis of PCR products from bisulfite-converted genomic DNA" ANALYTICAL BIOCHEMISTRY, vol. 354, no. 2, 6 May 2006 (2006-05-06), pages 266-273, XP002483856 ISSN: 0003-2697
- CORNELIUS MICHAEL ET AL: "Detection and separation of nucleoside-5 '-monophosphates of DNA by conjugation with the fluorescent dye BODIPY and capillary electrophoresis with laser-induced fluorescence detection" ELECTROPHORESIS, vol. 26, no. 13, June 2005 (2005-06), pages 2591-2598, XP002483857 ISSN: 0173-0835
- YANG INCHUL ET AL: "Rapid quantification of DNA methylation through dNMP analysis following bisulfite-PCR." NUCLEIC ACIDS RESEARCH 2006, vol. 34, no. 8, 2006, page e61, XP002467528 ISSN: 1362-4962
- THOMASSIN H.: 'MethylQuant: a sensitive method for quantifying methylation of specific cytosines within the genome' NUCLEIC ACIDS RESEARCH vol. 32, no. 21, 02 December 2004, page E168, XP003003354
- SADRI R.: 'Rapid analysis of DNA methylation using new restiction enzyme sites created by bisulfite modification' NUCLEIC ACIDS RESEARCH vol. 24, no. 24, 1996, pages 5058 - 5059, XP002192857

## Description

### Technical Field

The present invention relates to a method for quantitatively analyzing methyl cytosine in DNA, and more particularly to a method for quantitatively analyzing methyl cytosine in DNA, the method comprising treating DNA extracted from a sample with bisulfite, amplifying the treated DNA by PCR, cleaving the amplified product into a deoxynucleotide monophosphate (dNMP) level, and separating the cleaved DNA by capillary electrophoresis, to thereby quantify and measure the content of methyl cytosine in a target gene.

### Background Art

Generally, methyl cytosine is found primarily in CpG dinucleotide present in the promoter of a gene, and the expression of the relevant gene is suppressed as a result of methylation. DNA methylation mediates the normal development of an organism by temporally and spatially delicately regulating the expression of a specific gene during the development of the organism. Thus, it is known that failure to regulate DNA methylation causes various negative results such as various developmental disorders, fetal death, tumorigenesis, aging and degenerative diseases, in an individual.

Also, as the fact that DNA methylation has a close connection with the development and progression of various diseases, including cancer, is found, studies to use DNA methylation as a marker for diagnosing various diseases and predicting the prognosis thereof are actively ongoing. It was found in various studies that genes such as P16INK4a, BRCA1, hMLH1 and E-cadherin are abnormally methylated in lung cancer, colon cancer and breast cancer, and it is known that the methylation of genes such as uPA, Caspase 9 and E- cadherin leads to a remarkable reduction in the effect of chemotherapy for the relevant cancer.

Since the methylation or unmethylation of a specific gene gives information on the progression and prognosis of the relevant disease as described above, studies to use the information of DNA methylation as a disease marker or prognosis marker have recently been actively conducted.

As the biological and clinical importance of DNA methylation thus increases, technologies for analyzing DNA methylation patterns also rapidly advance. Several standards for the classification of a variety of DNA methylation analysis methods, which have been developed so far, include: (1) whether a target is total DNA in the genome or only a specific target gene; (2) whether a specific gene is analyzed for all of a few ten methylatable CpGs or only one or two CpGs; and (3) whether methylation analysis is quantitative or qualitative. In view of these standards, the following four requirements must be satisfied in order for the analysis of DNA methylation to be conveniently used in clinical trials:
1. Analysis of a specific target gene
2. Analysis of methylation of all CpGs in a given DNA fragments
3. Quantitative analysis
4. Convenience and economy of analysis

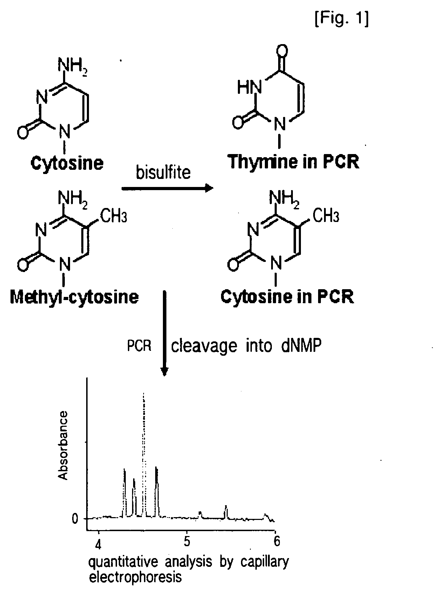

Although about 20 kinds of methylation analysis methods have been developed so far (Laird, P.W., Nature Rev. Cancer, 3, 253-266, 2003), any of the developed methods does not satisfy the above-described requirements such that it can be actually used in clinical trials. For example, an HPLC or TLC method is easily used to quantitatively analyze the amount of methyl cytosine in the total genome, but has shortcomings in that it requires a large amount of a sample and cannot perform analysis for a specific gene. Also, a Southern blot method using a methylation sensitive restriction enzyme can perform quantitative analysis for a specific gene, but has shortcomings in that it can analyze the methylation or unmethylation of only one CpG among a few ten CpGs of a specific gene fragment, and requires a time of at least three days for one performance, and thus has a low economic efficiency of analysis. Among the various developed methods, the most widely applied method is a method using bisulfite. In this method, when DNA is treated with a high concentration (about 3-5 moles) of bisulfite and subjected to desulfonation, cytosine will not undergo any change, but unmethylated cytosine will be chemically converted into uracil (see FIG. 1). Also, any of the currently developed methods cannot amplify a specific gene while maintaining the location and amount of methyl cytosine, however, when the bisulfite method is applied, a "methyl cytosine/unmethylated cytosine" relationship will be replaced with a "cytosine/uracil (or thymine)" relationship, in which a specific gene can be amplified while maintaining information on the location and amount of methyl cytosine. In other words, the process comprising bisulfite treatment and PCR is a method that selectively amplifies only a specific gene while maintaining the location and amount of methylation. This process has advantages in that it not only allows difficult-to-analyze DNA modification information to be converted into easy-to-analyze base sequence information, but also makes the interpretation of analysis results clear, because the required amount of a sample is reduced and the methylation information of other genes is excluded, due to the selective amplification of a specific gene. For this reason, most of recent methylation analysis methods are frequently based on the bisulfite treatment and PCR amplification process (see Table 1).

**(Table 1)**

| Analysts method | Principle | Specificity | Sensitkivity | Multi CpG | Quantification possibility | Economic efficiency |
|---|---|---|---|---|---|---|
| Anti-mC-antibody | Anti-mC-an tibody | Global | L | Y | Y | M |
| HPTC-UV/LIF | HPLC | Global | L/H | Y | Y | H/L |
| Southern blot | MS-RE | specific | L | N | Y | L |
| MS-SNuPE | Bisulfite conv. | Specific | M | N | Y | M |
| COBRA | Bisulfite conv. | Specific | H | N | Y | H |
| PyroMeth | Bisulfite conv. | Specific | M | Y | Y | M |
| Bisulfite seq. (clones) | Bisulfite conv. | Specific | H | Y | Y | L |
| MSP | Bisulfite conv. | Specific | H | N/Y | N/Y | H |
| Bisulfite-DGGE | Bisulfite conv. | Specific | H | Y | Y | H |
| MethyLight | Bisulfite conv. | Specific | H | N/Y | Y | M |
| Bisulfite- SSCP | Bisulfite conv. | Specific | H | N/Y | Y | M |
| MSP/DHPLC | Bisulfite conv. | Specific | H | Y | Y | M |
| RLGS | MS-RE | Screening | M | N | Y | L |
| MBD | MBD | Screening | M | Y | N/Y | M |
| MS-AP-PCR | MS-RE | Screening | H | N | Y | M |
| MCA | MS-RE | Screening | M | N | N/Y | M |
| DMH | MS-RE | Screening | M | N | N/Y | M |
| MS-microarr ay | Bisulfite conv. | Screening | H | N/Y | Y | M |

| | | | | | | |
|---|---|---|---|---|---|---|
| H: high; M: medium; L: low; H/L: high in special cases; Y: possible; N: not possible; N/Y: partially possible | | | | | | |

However, some of the methods listed in Table 1 above are frequently used in operations for finding abnormally methylated genes. For example, the RLGS method or MS-AP-PCR method is a technology suitable to screen genes having more or less methylation in any one of two control groups, at the total genome level. On the other hand, several analysis methods, which are conducted after bisulfite treatment and PCR reaction, are frequently used to analyze the methylation or unmethylation of a specific gene at high speed and high sensitivity, when the target gene was determined.

Examples of these analysis methods include MS-PCR, COBRA, MethyLight.

However, these methods can perform a high-speed and high-sensitivity analysis, but have a disadvantage in that, because only the methylation or unmethylation of one or two CpGs is mostly analyzed, the representativeness of analysis and quantification is slightly reduced. Thus, in most cases, validation analysis is additionally required after primary analysis was performed using the above-mentioned methods.

Furthermore, the only final validation method, which has currently been developed, is a bisulfite treatment-cloning-nucleotide sequencing method. This method is used to validate the methylation or unmethylation of all CpG sites in a PCR product by treating a specific gene with bisulfite, amplifying the treated gene by PCR, cloning the PCR products into individual clones and analyzing the base sequence of each of the clones. Generally, based on base sequence analysis results for about 20 clones, the methylation or unmethylation of the relevant gene is typically indicated. Although the bisulfite treatment-cloning-nucleotide sequencing method is now the best methylation analysis and validation method, it is disadvantageous in terms of time and economic efficiency, because it requires cloning and base sequence analysis operations for a number of clones.

The following prior art references relate to methods for quantitative DNA methylation analysis which differ in conduct and properties from the present method:

FRAGA M F ET AL: "High-performance CE method for the quantification of 5-mC in genomic DNA: application to plant, animal and human cancer tissues." ELECTROPHORESIS, 2002, vol. 23, no.11, pages 1677-1681, ISSN: 0173-0835.

FRANK LYKO: "Novel methods for analysis of genomic DNA methylation" ANALYTICAL AND BIOANALYTICAL CHEMISTRY, vol. 381, no. 1, 1 January 2005, pages 67-68, ISSN: 1618-2650.

FRAGA M F ET AL: "DNA METHYLATION: A PROFILE OF METHODS AND APPLICATIONS" BIOTECHNIQUES, vol. 33, no. 3, 1 September 2002, pages 632,634,636-649, ISSN: 0736-6205.

CHRISTINA DAHL ET AL: "DNA methylation analysis techniques" BIOGERONTOLOGY, vol. 4, no. 4, 1 August 2003, pages 233-250.

YANG INCHUL ET AL: "Rapid quantification of DNA methylation through dNMP analysis following bisulfite-PCR." NUCLEIC ACIDS RESEARCH, 2006, vol. 34, no. 8, page e61, ISSN: 1362-4962.

### Disclosure of Invention

### Technical Problem

The present inventors have conducted studies to develop a new technology for quantitatively analyzing DNA methylation, which can overcome the shortcomings of a large number of DNA methylation analysis methods introduced so far, in that, when quantification capability is good, it is difficult to analyze an individual gene, and when the analysis of the individual gene is easy and economic efficiency is high, quantification capability is reduced, the new technology making it possible to analyze an individual gene level and to obtain quantitative information for total methylation sites in a gene.

Accordingly, it is an object of the present invention to provide a method for quantitatively analyzing the content of methyl cytosine in a target gene by treating genomic DNA extracted from an sample with bisulfite, amplifying the target gene in the treated DNA by PCR, and quantitatively measuring the content of cytosine in the amplified gene by capillary electrophoresis.

Another object of the present invention is to provide a method of using said quantitative analysis method to classify and diagnose aging, cancer, pathogens, pathogenic contamination, tissues or individuals.

### Technical Solution

To achieve the above objects, the present invention provides a method for quantitatively analyzing the methylation levels in a target gene, the method comprising the steps of:
(1) treating genomic DNA extracted from a cell or tissue sample with bisulfite so as to maintain methyl cytosine intact and to convert unmethylated cytosine into uracil;
(2) selectively amplifying the target gene by PCR using the DNA treated in said step (1) as a template in a reaction solution containing primers specific for the target gene, dNTP and polymerase;
(3) removing non-specific amplification products, the dNTP and the primers from the product amplified in said step (2) to purify the target gene;
(4) cleaving the target gene purified in said step (3) into a deoxynucleotide monophosphate (dNMP) level;
(5) separting the dNMP-level product cleaved in said step (4) using capillary electrophoresis, to thereby quantify and measure the C/T and G/A ratios in the target gene; and
(6) validating or calibrating the C/T and G/A ratios in the target gene quantified and measured in step (5), referring to the C/T and G/A ratios in an oligo DNA having a known base sequence, as a standard for quantification, quantified and measured according to steps (1) through (5), wherein the measured C/T and G/A ratios for the oligo DNA are divided by the values calculated from its base sequence, and the obtained values are used as calibration coefficients to calibrate the measured C/T and G/A ratios in the target gene.

In another aspect, the present invention provides a method for classifying or diagnosing aging, cancer, pathogens, pathogenic contamination, tissues and individuals in a sample, the method comprising measuring the methylation level in the sample using said methyl cytosine quantitative analysis method, and comparing the measured content with the database content of methyl cytosine for aging, cancer, pathogens, tissues or individuals.

A basis on which cancer in said diseases can be diagnosed according to the present invention is supported by the following known references on cancers caused by the methylation of target genes:
E-cadherin involves point mutation or promoter hypermethylation; abnormalities of e-cadherin include acute leukemia (70% frequency), breast cancer (30-75%), esophageal cancer (84%), liver cancer (40-70%), and ulcerative colitis-induced colon cancer (40-70%), and in the cases of these cancers, the examination of mutation of e-cadherin becomes an useful assistant diagnosis method (Guliford, P. J et al. Hum. Mutat, 1999. 14: 249-255, 1999; Machado JC et al. Oncogene, 2001. 20(12):1525-1528; Droufakou S et al. Int J Cancer, 2001, 92(3):404-408).

The promoter CpG island methylation of FHIT (fragile histidine triad) is known as a good diagnostic marker in lung cancer, head and neck cancer, esophageal cancer and the like (Inoue, H et al. Proc. Nat. Sci, 1997. 94:12584-14589; Sozzi G et al. Cancer Res,1998. 58:5032-7; Zochbauer-Muller Set al Cancer Res, 2001).

The examination of promoter methylation of the P16 gene can be used to diagnose a number of cancers, including lung cancer, head and neck cancer, liver cancer, and bile duct cancer, and the P15 gene is useful for the diagnosis of acute leukemia and gastric cancer (Esteller M et al. Cancer Res, 1999. 59:67-70;Melki JR et al. Cancer RES, 2999. 59:3730-40;Liew CT et al. Oncogene, 1999. 18:789-93; Wong IH et al. Clin Cancer RES, 2000. 6:3516-21; Kersting M. J Clin Oncol, 2000. 18:3221-4; Rosas SL et al. Cancer RES, 2001. 61:939-42).

MGMT (methylguanine-DNA methyltransferase) can be used as a marker for the diagnosis of glioma, colon cancer and lung cancer due to promoter hypermethylation, and a method of diagnosing lung cancer and head and neck cancer by examining the promoter hypermethylation of MGMT in blood, phlegm and saliva is known in America and Europe (Esteller M et al. Cancer Res, 1999. 59:793-797;Esteller M et al. Cancer Res, 2001. 61:4689-4692; Esteller M et al. Cancer Res, 2001. 61:3225-9).

In addition, it is well known that the hypermethylation of a gene promoter suppresses the expression of the relevant gene to cause cancer or promote aging.

Hereinafter, the present invention will be described in detail.

Unless otherwise defined, all technical and scientific terms used herein have the same meaning as commonly understood by one of ordinary skill in the art to which the present invention pertains.

Also, the repeated description for the same technical construction and operation as in the prior art will be omitted herein.

The present invention relates to a method for quantitatively analyzing the degree of methylation in an individual gene and utilizes a characteristic in that, if DNA is treated with bisulfite, cytosine will be converted into uracil, but methyl cytosine will remain as methyl cytosine intact, and if a gene to be analyzed is amplified by PCR, the PCR product will contain thymine at a general cytosine location and cytosine at a methyl cytosine location. Using this characteristic, the content of methyl cytosine in a target gene is quantitatively analyzed by treating genomic DNA extracted from a sample with bisulfite, amplifying the target gene in the treated DNA by PCR, and quantitatively measuring the ratio of cytosine in the amplified target gene by capillary electrophoresis.

A sample, which is used in the present invention, is preferably DNA extracted from a sample selected from the group consisting of cells, blood, salvia, feces, urine, serum, skin, eye, intestines, kidneys, brain, heart, prostate, lungs and liver. This is because said samples are suitable to classify and diagnose aging, cancer, pathogens, pathogenic contamination, tissues or individuals, according to the object of the present invention.

Also, in order to quantify the content of methyl cytosine in the sample, the cleavage into dNMP in the step (4) of the present invention is preferably performed using a biological enzymatic cleavage method that uses an enzyme selected from the group consisting of nuclease, DNase and a mixture thereof, a chemical cleavage method comprising acid or base treatment or heating, and a physical cleavage method that uses a cleavage device selected from the group consisting of an ultrasonic wave generator, a shearing wave generator and a particle accelerator.

The amount of sample used can be changed depending on the content of DNA, and the treatment of the sample can be performed according to any method known in the art. This is obvious to any person skilled in the art to which the present invention pertains.

Also, the dNMP in the step (5) of the present invention is preferably quantified using a measurement device selected from the group consisting of high-performance liquid chromatography (HPLC), liquid chromatography/mass spectrometry (LC/MS), thin-layer chromatography (TLC) and gas chromatography (GC). This is because the above-described devices can sufficiently detect the DNA base sequences separated by capillary electrophoresis even at low contents (initial concentration: 1□/50□).

Each step of the inventive method for quantitatively analyzing methyl cytosine in a gene has the following characteristics.

### (1) Bisulfite treatment step

This step is a step of treating genomic DNA to be analyzed, with bisulfite, to convert unmethylated cytosine into uracil, according to a conventional method known in the art. Preferably, the treatment with bisulfite is conducted using 4M bisulfite at a pH of 4.0-5.0 at a temperature of 50-60 °C for 4-5 hours. This is because, near said bisulfite concentration, DNA is stable in bisulfite treatment and is advantageous for reaction. Also, the bisulfite concentration or treatment time is not limited as long as it can achieve the above object.

The genomic DNA to be analyzed is first separated into single strands in a strong base condition.

### (2) Step of amplifying target gene by PCR

This step is a step of amplifying a specific gene for analysis by PCR using the bisulfite-treated DNA as a template and isolating the amplified product. This step is performed in a reaction buffer mixture containing primers specific for the target gene, DNA polymerase and dNTP, according to a conventional PCR method known in the art.

### (3) Step of purifying target gene of amplified product

The target gene is purified by removing non-specific amplification products, the dNTP and the primer. Preferably, a commercially available purification kit is used in order to minimize an error in the content of methyl cytosine in the target gene during capillary electrophoresis.

### (4) Cleavage of purified target gene into dNMP level

The amplified and isolated target gene (PCR product) is cleaved into the level of dNMP, a DNA monomer, using a biological method that uses an enzyme, a chemical method that uses acid or base treatment or heating, or a physical method that uses ultrasonic wave or shearing wave.

### (5) Step of separating cleaved dNMP by capillary electrophoresis and quantitatively analyzing the separated fragments

In order to validate or calibrate the quantitatively measured value for methyl cytosine, the present invention further comprises a step of calibrating the quantitative result of the step (5) using a suitable quantitative reference (oligo DNA having a known base sequence) so as to finally quantify the degree of methylation of the target gene.

Hereinafter, the present invention will be described in further detail with reference to the accompanying drawings. However, it will be obvious to a person skilled in the art that these drawings are presented to aid the understanding of the present invention and are not intended to limit the scope of the present invention.

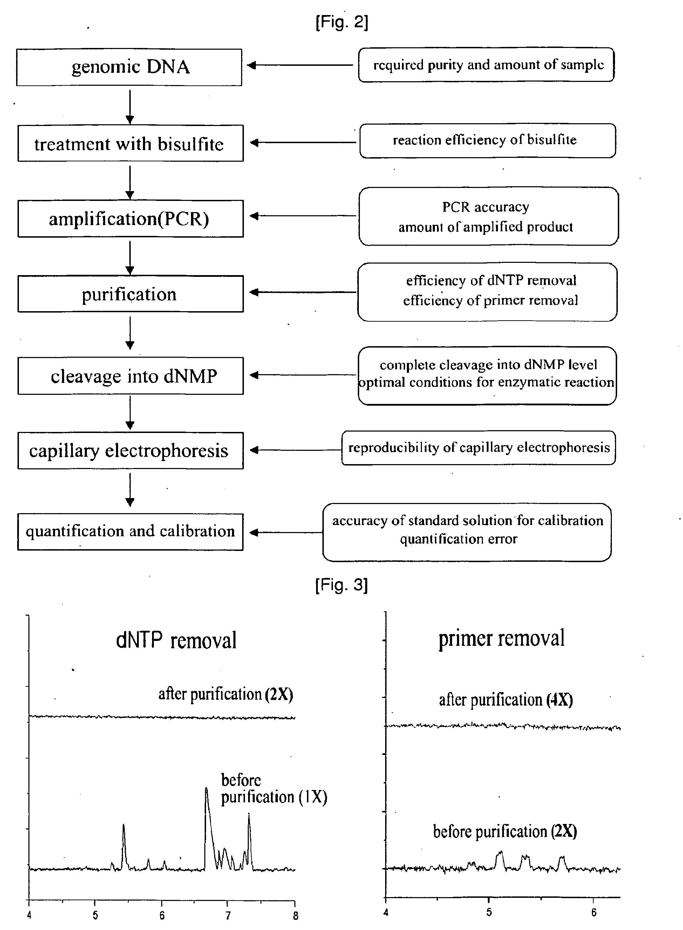

FIG. 2 is a flow chart showing the sequential steps of a quantitative analysis process according to the present invention. In addition, details to be validated in order to increase the accuracy of quantitative analysis and to minimize unreliability are shown for each step in FIG. 2. The inventive process for analyzing DNA methylation consists essentially of the above-described five steps.

As shown in FIG. 1, when DNA is treated with bisulfite, the deamination of cytosine in the DNA will occur so that cytosine and methyl-cytosine are distinguishable from each other, and it is generally known that a conversion reaction with bisulfite has an efficiency of more than 97%. Particularly, it is known that the use of a low-melting temperature agarose method, which was recently developed, or a commercially available bisulfite reaction kit (CpGenome kit, Chemicon, Temecula, CA, USA; EZ DNA methylation kit, Zymo Research, Orange, CA, USA), can provide a conversion efficiency of more than 99%. The bisulfite conversion is followed by a step of amplifying an individual gene by PCR and isolating and purifying the amplified product. In the present invention, in order to prevent non-specific amplification products from being produced in PCR amplification, it is preferable to conduct PCR amplification once more using a nested PCR primer. The amplified PCR product contains the primer and dNTP added in the start of the PCR reaction, which are compounds that can be converted into dNMP to be subsequently analyzed in the present invention. In the present invention, in order to effectively remove the remaining primer and dNTP, a commercial PCR purification kit (Qiagen, Hilden, Germany) was used for purification. Then, a step of cleaving the purified PCR product into dNMP to be analyzed in capillary electrophoresis was performed using phosphodiesterase (AmershamBiosciences, Piscataway, NJ, USA) in the present invention. Then, the reaction product cleaved into the dNMP level was immediately separated and quantitatively analyzed using capillary electrophoresis (P/ACE MDQ CE system, Beckman Coulter, Fullerton, CA, USA) without subjecting to a separate purification process. In the capillary electrophoresis process, the relative ratio of cytosine to thymine, but not the absolute amount of cytosine, was quantitatively analyzed.

According to the principle of analysis for methyl cytosine, the measurement of amount of cytosine makes it possible to determine the amount of methyl cytosine in original DNA. However, the actual PCR product consists of double strands, and thus, when guanine is present in one strand, cytosine inevitably follows in the opposite strand. Accordingly, in the present invention, the final ratio of methyl cytosine is determined by subtracting a ratio caused by guanine in the opposite strand according to the base sequence of a target gene from the relative ratio of cytosine calculated in the capillary electrophoresis.

In the present invention, it is preferable to use a synthetic oligo-primer having an already known base sequence, as a standard reference for quantification, instead of making a reference for quantification by gravimetric determination. This is a first attempt in Korea, because a precise quantitative reference for dNMP has not existed. Herein, an amount to be analyzed in the present invention is the cytosine/thymine ratio, but not the absolute amount of cytosine, and thus, as long as the ratio of cytosine to thymine present in a single molecule is exact, the molecule can be used as a reference for quantification. However, because the quantitative reference has low purity and can contain other molecules, an oligo-primer purely isolated using polyacrylamide gel is analyzed according to the same process, and used as a reference to calibrate analyzed values.

### Brief Description of the Drawings

FIG. 1 is a drawing showing that methyl cytosine is changed into a form distinguishable from unmethylated cytosine by bisulfite reaction and PCR reaction;
FIG. 2 schematically shows a process for quantitatively analyzing methyl cytosine according to the present invention;
FIG. 3 shows that dNTP and primers, which interfere with analysis, are removed by the purification of a PCR product.
FIG. 4 shows the results of capillary electrophoresis conducted in a process for quantitatively analyzing DNA fragments according to a method of one preferred embodiment of the present invention, after the distribution and amount of methyl cytosine in the DNA fragments are analyzed by bisulfite sequencing.

### Mode for the Invention

### Example

### (1) Treatment with bisulfite

In the present invention, two kinds of DNAs were treated with bisulfite and used in analysis. As one DNA among these DNAs, simple plasmid DNA (pUC19; NEB, Beverly, MA, USA) was used to validate a principle and procedure after it was artificially methylated, and as the other DNA, genomic DNA extracted from a liver cancer cell line was used to validate the applicability of the developed method. The artificial methylation of the plasmid DNA was performed using Sss I methylase (NEB) and Hha I methylase. The bisulfite treatment was conducted in the following manner. About 1 □/50□ of genomic DNA cut to a suitable size with restriction enzymes was separated into single strands by treatment with 0.3M NaOH for 15 minutes, and then mixed with 500 □ of a freshly prepared solution containing 4M sodium bisulfite (Sigma, USA) and 1 mM hydroquinone (pH 5.0), and the upper layer was covered with mineral oil. The reaction solution was covered with a lid such that light could not be passed into the solution, and then was allowed to react at 55 °C for 4 hours. After completion of the reaction, the reaction product was purified using Microcon YM 30 (Millipore, Bedford, MA, USA) column and allowed to react with 0.3 M NaOH at 37 °C for 30 minutes to induce desulfonation. The reaction product was purified again using the Microcon YM 30 column and recovered using 50 □ of triple-distilled water. 2 □ of the recovered DNA was used as a template in one-time PCR.

### (2) PCR amplification, isolation and purification

The following PCR primers were used to amplify a DNA region of about 300 bp from the plasmid DNA treated with bisulfite: pl9bs (forward): AAGTG-TAAAGTTTGGGGTGTTTAA; p19bs (reverse): AACCTTTTACTCACATATTCTT TCCTAC. The amplified PCR product was purified using a PCR purification kit (Qiaquick; Qiagen), and it was confirmed on agarose gel that the purified DNA did not contain other kinds of DNAs. In the purification of the PCR product, 100 □ of the PCR reaction solution was recovered in an amount of 30 □, such that the PCR product was concentrated to a volume of about 1/3 after purification. To analyze methylation by sequencing, some of the PCR product was cloned into a pCRSCRIPT II (Stratagene, La Jolla, CA, USA) vector. Each of the clones was sequenced in Genotech (Daejeon, Korea).

### (3) Cleavage of PCR product and quantitative analysis by capillary electrophoresis

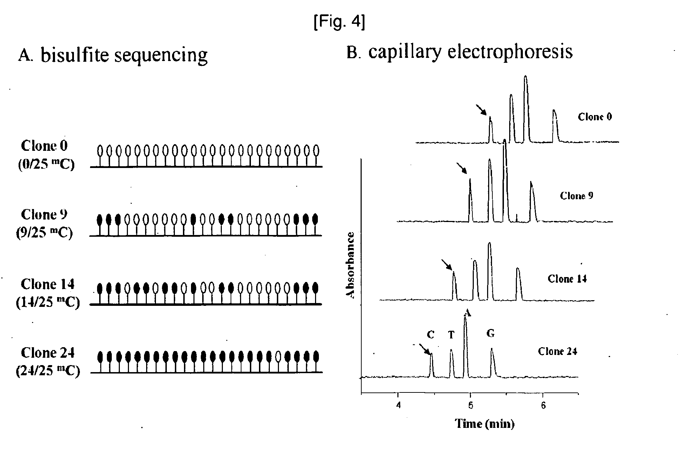

The purified DNA was added to 0.05 units of phosphodiesterase in PCR buffer conditions, and allowed to react at 37 °C for 1 hour so that it was completely cleaved into the 5'-dNMP level. After completion of the reaction, the reaction product was left to stand at 80 °C for 15 minutes in order to inactivate the enzyme. When the PCR product is not purified, peaks that can interfere with quantitative results due to the primers or dNTP mixture used in the PCR reaction can be produced in capillary electrophoresis. For this reason, the PCR product was analyzed before and after purification and, as a result, it was confirmed that the remaining primers or dNTPs were completely removed after purification (see FIG. 3). Immediately after cleaving the reaction product into the dNMP level, the dNMPs were separated and quantitatively analyzed using a capillary electrophoresis system without a separate purification process. In the separation of the dNMPs, a melted silica capillary column (Polymicro Technology, Phoenix, AZ, USA) having a total length of 70 cm (length to detector: about 60 cm), an inner diameter of 50 □ and an outer diameter of 365 □ was used, and as a solvent for separation, a buffer solution (pH 10.1) consisting of 8mM cetyltrimethylammonium bromide (CTAB), 100 mM 2-amino-2-methyl-1-propanol (AMP) and 46 mM NaCl was used. The injection of the sample was conducted using an electrodynamic method at -5 kV for 10 seconds, and the electrophoresis of the sample was conducted at -20 kV and 25 °C. Each of molecules was detected at UV 260 nm. FIG. 4 shows the results of capillary electrophoresis conducted for DNA whose methylation degree was determined by bisulfite sequencing, in which the capillary electrophoresis was conducted according to the process developed in the present invention.

### (4) Calibration of quantitative results for cytosine/thymine and final methylation quantification

An amount to be quantified in capillary electrophoresis is the relative amount of cytosine/thymine, but not the absolute amount of cytosine. Thus, as a reference for quantification, pure oligo-DNA having a known base sequence was used instead of pure dNMP prepared by gravimetric determination. As the reference oligos, the following oligos purely isolated through polyacrylamide gel were used. T7: TAATAC-GACTCACTATAGGG; and P16r: GTCTGCTGAAACTGCCAACA. The T7 oligo must be measured at a C/T ratio of 1 and a G/A ratio of 0.571 in a base sequence, and the P16r oligo must be measured at a C/T ratio of 1.5 and a G/A ratio of 0.5. Because dNMPs have different UV absorbance coefficients, the result quantified only with the area of a peak in capillary electrophoresis is different from the actual ratio of each molecule. Thus, the reference oligos were measured, the C/T and G/A ratios measured in each of the oligos were divided by a value calculated for a base sequence, and the obtained values were used as calibration coefficients to calibrate quantitative results for actual methylation analysis samples. According to the above-described method, the methylation degrees of the plasmid DNAs, which have been methylated in manners different from each other, were quantified and compared with analysis results for base sequences, and the results are shown in Table 2 below.

| Clones | C0 | | C9 | | C14 | | C24 | |
|---|---|---|---|---|---|---|---|---|
| | C/T | G/A | C/T | G/A | C/T | G/A | C/T | G/A |
| Calculated | 0.431 | 0.431 | 0.503 | 0.503 | 0.543 | 0.54.3 | 0.624 | 0.624 |
| Measured | 0.371± | 0.416± | 0.440± | 0.482± | 0.468± | 0.528± | 0.548± | 0.607± |
| | 0,011 | 0.002 | 0.015 | 0.016 | 0.014 | 0.021 | 0.018 | 0.018 |
| Calibration | 0.418 | 0.431 | 0.495 | 0.500 | 0.527 | 0.541 | 0.617 | 0.629 |
| Difference | 2.948 | -0.043 | 1.514 | 0.644 | 2.966 | -0.718 | 1.104 | -0766 |
| (%) | | | | | | | | |
| Difference | 1.605 | -0.023 | 0.962 | 0.409 | 2.034 | -0.492 | 0.870 | -0.603 |
| (#"'C) | | | | | | | | |

In Table 2 above, "calculated" represents analysis results for base sequences treated with bisulfite, "measured" represents the results of measurement by capillary electrophoresis, calibration represents the results of calibration with calibration coefficients, "difference(%)" represents the difference of the calibration value from the calculated value, and "difference (#^{m}C)" represents a difference in the number of methyl cytosines, which corresponds to the difference between a measured value among 25 methylatable CpGs and a value obtained by quantitative analysis in this Example.

From the above results and the predicted comparison, it can be seen that, according to the method for the quantification of methylation, developed in the present invention, the degree of methylation in a specific DNA fragment can be quantified within an error of about 5%. This suggests that, if 20 methylated CpGs are actually present in a specific DNA fragment, the method presented in the present invention will predict that 19-21 methylated CpGs are present in the specific DNA fragment.

### Industrial Applicability

As described above, the inventive method for the quantitative analysis of DNA methylation enables the degree of DNA methylation to be quantified in a high-speed, high-sensitivity and automatable platform, which has not yet been achieved by any of the prior methods. In the present invention, the time required to quantify one sample is about 7 hour when started with genomic DNA, and if the bisulfite treatment and PCR amplification processes, which are also conducted in most of other methylation analysis methods, are not considered, the inventive method requires about 1 hour for cleavage into dNMPs, and about 10 minutes for capillary electrophoresis. This can be regarded as an unprecedented high-speed method for the quantification of methylation, and because all the processes, including PCR amplification, purification, cleavage into the dNMP level, and capillary electrophoresis, can treat several tens of samples at the same time, the time required for an individual sample in carrying out large-scale quantitative analysis can be significantly reduced.

Also, the error of quantification presented in Example of the present invention is less than 5 %, which can be further reduced through the optimization of capillary electrophoresis conditions. Thus, it is believed that, when the present invention is applied in the art to which the invention pertains, it will receive a great deal of attention

## Claims

1. A method for quantitatively analyzing the methylation levels in a target gene, the method comprising the steps of:
(1) treating genomic DNA extracted from a cell or tissue sample with bisulfite so as to maintain methyl cytosine intact and to convert unmethylated cytosine into uracil;
(2) selectively amplifying the target gene by PCR using the DNA treated in said step (1) as a template in a reaction solution containing primers specific for the target gene, dNTP and polymerase;
(3) removing non-specific amplification products, the dNTP and the primers from the product amplified in said step (2) to purify the target gene;
(4) cleaving the target gene purified in said step (3) into a deoxynucleotide monophosphate (dNMP) level;
(5) separating the dNMP-level product cleaved in said step (4) using capillary electrophoresis, to thereby quantify and measure the C/T and G/A ratios in the target gene; and
(6) validating or calibrating the C/T and G/A ratios in the target gene quantified and measured in step (5), referring to the C/T and G/A ratios in an oligo DNA having a known base sequence, as a standard for quantification, quantified and measured according to steps (1) through (5), wherein the measured C/T and G/A ratios for the oligo DNA are divided by the values calculated from its base sequence, and the obtained values are used as calibration coefficients to calibrate the measured C/T and G/A ratios in the target gene.

2. The method of Claim 1, wherein the sample is DNA extracted from a sample selected from the group consisting of cells, blood, salvia, feces, urine, serum, skin, eye, intestines, kidneys, brain, heart, prostate, lungs and liver.

3. The method of Claim 1, wherein the cleavage in said step (4) is performed using an enzyme selected from the group consisting of nuclease, DNase and a mixture thereof.

4. The method of Claim 1, wherein the cleavage in said step (4) is performed using a chemical process selected from among acid treatment, base treatment or heating.

5. The method of Claim 1, wherein the cleavage in said step (4) is performed using a cleavage device selected from the group consisting of an ultrasonic wave generator, a shearing wave generator and a particle accelerator.

6. The method of Claim 1, wherein the dNMP in said step (5) is quantified using a measurement device selected from the group consisting of high-performance liquid chromatography (HPLC), liquid chromatography/mass spectrometry (LC/MS), thin-layer chromatography (TLC) and gas chromatography (GC).

7. An analysis method for classifying or diagnosing aging, cancer, pathogens, pathogenic contamination, tissues and individuals in a sample, the method comprising measuring the methylation level in the sample using the quantitative analysis method of any one of Claims 1 to 6, and comparing the measured content with the database content of methyl cytosine for aging, cancer, pathogens, tissues or individuals.

## Patentansprüche

1. Verfahren zum quantitativen Analysieren der Methylierungslevel in einem Targetgen, wobei das Verfahren die folgenden Schritte umfasst:
(1) Behandeln von genomischer DNA, die aus einer Zell- oder Gewebeprobe extrahiert wurde, mit Bisulfit, um so Methylcytosin intakt zu halten und unmethyliertes Cytosin in Uracil umzuwandeln;
(2) selektives Amplifizieren des Targetgens durch PCR unter Verwendung der in Schritt (1) behandelten DNA als Matrize in einer Reaktionslösung, die für das Targetgen spezifische Primer, dNTP und Polymerase enthält;
(3) Entfernen von nicht-spezifischen Amplifikationsprodukten, von dNTP und der Primer von dem in Schritt (2) amplifizierten Produkt, um das Targetgen zu reinigen;
(4) Spalten des in Schritt (3) gereinigten Targetgens zu einem Desoxynucleotidmonophosphat-(dNMP)-Level;
(5) Abtrennen des in Schritt (4) gespaltenen dNMP-Level-Produkts unter Verwendung von Kapillarelektrophorese, um **dadurch** die C/T- und G/A-Verhältnisse in dem Targetgen zu quantifizieren und zu messen, und
(6) Validieren oder Kalibrieren der C/T- und G/A-Verhältnisse in dem Targetgen, die in Schritt (5) quantifiziert und gemessen wurden, unter Bezug auf die C/T- und G/A-Verhältnisse in einer Oligo-DNA mit einer bekannten Basensequenz als Standard zur Quantifizierung, die nach den Schritten (1) bis (5) quantifiziert und gemessen wurden, wobei die gemessenen C/T- und G/A-Verhältnisse für die Oligo-DNA durch die aus ihrer Basensequenz errechneten Werte dividiert werden und die erhaltenen Werte als Kalibrierungskoeffizienten verwendet werden, um die gemessenen C/T- und G/A-Verhältnisse in dem Targetgen zu kalibrieren.

2. Verfahren gemäß Anspruch 1, wobei die Probe DNA ist, die aus einer Probe extrahiert wurde, welche aus der Gruppe, bestehend aus Zellen, Blut, Speichel, Fäzes, Urin, Serum, Haut, Auge, Darm, Nieren, Gehirn, Herz, Prostata, Lungen und Leber, ausgewählt wird.

3. Verfahren gemäß Anspruch 1, wobei die Spaltung in Schritt (4) unter Verwendung eines Enzyms durchgeführt wird, das aus der Gruppe, bestehend aus Nuclease, DNase und einem Gemisch davon, ausgewählt wird.

4. Verfahren gemäß Anspruch 1, wobei die Spaltung in Schritt (4) unter Verwendung eines chemischen Verfahrens durchgeführt wird, das aus Säurebehandlung, Basenbehandlung und Erwärmen ausgewählt wird.

5. Verfahren gemäß Anspruch 1, wobei die Spaltung in Schritt (4) unter Verwendung einer Spaltungsvorrichtung durchgeführt wird, die aus der Gruppe, bestehend aus einem Ultraschallwellengenerator, einem Scherwellengenerator und einen Partikelbeschleuniger, ausgewählt wird.

6. Verfahren gemäß Anspruch 1, wobei das dNMP in Schritt (5) unter Verwendung einer Messvorrichtung, ausgewählt aus der Gruppe, bestehend aus Hochleistungsflüssigkeitschromatografie (HPLC), Flüssigkeitschromatografie/Massenspektrometrie (LC/MS), Dünnschichtchromatographie (TLC) und Gaschromatographie (GC), ausgewählt wird.

7. Analyseverfahren zur Klassifizierung oder Diagnostizierung von Altern, Krebs, Pathogenen, pathogener Kontamination, Geweben und Individuen in einer Probe, wobei das Verfahren Messen des Methylierungslevels in einer Probe unter Verwendung des quantitativen Analyseverfahrens gemäß einem der Ansprüche 1 bis 6 und Vergleichen des gemessenen Gehalts mit dem Datenbanken-Gehalt an Methylcytosin für Altern, Krebs, Pathogene, Gewebe oder Individuen umfasst.

## Revendications

1. Procédé destiné à analyser de manière quantitative les taux de méthylation dans un gène cible, le procédé comprenant les étapes consistant à :
(1) traiter un ADN génomique extrait d'un échantillon de cellules ou de tissus avec du bisulfite de sorte à maintenir intacte la méthyl cytosine et à convertir la cytosine non méthylée en uracile ;
(2) amplifier de manière sélective le gène cible par PCR en utilisant l'ADN traité à ladite étape (1) comme matrice dans une solution de réaction contenant les amorces spécifiques au gène cible, le dNTP et la polymérase ;
(3) éliminer les produits d'amplification non spécifiques, le dNTP et les amorces du produit amplifié à ladite étape (2) afin de purifier le gène cible ;
(4) couper le gène cible purifié à ladite étape (3) afin d'obtenir un produit de type désoxynucléotide monophosphate (dNMP) ;
(5) séparer le produit de type dNMP coupé à ladite étape (4) en utilisant l'électrophorèse capillaire, afin de quantifier et de mesurer de ce fait les rapports C/T et G/A dans le gène cible ; et
(6) valider ou calibrer les rapports C/T et G/A dans le gène cible quantifiés et mesurés à l'étape (5), en se référant aux rapports C/T et G/A dans un oligo-ADN ayant une séquence de bases connues, comme standard pour la quantification, quantifiés et mesurés selon les étapes (1) à (5), dans lequel les rapports C/T et G/A mesurés pour l'oligo-ADN sont divisés par les valeurs calculées à partir de sa séquence de bases, et les valeurs obtenues sont utilisées comme coefficients de calibration pour calibrer les rapports C/T et G/A mesurés dans le gène cible.

2. Procédé selon la revendication 1, dans lequel l'échantillon est de l'ADN extrait d'un échantillon choisi dans le groupe comprenant des cellules, du sang, de la salive, des selles, de l'urine, du sérum, de la peau, un oeil, de l'intestin, du rein, du cerveau, du coeur, de la prostate, des poumons et du foie.

3. Procédé selon la revendication 1, dans lequel la coupure à ladite étape (4) est réalisée en utilisant une enzyme choisie dans le groupe comprenant une nucléase, une DNase ou un mélange de ceux-ci.

4. Procédé selon la revendication 1, dans lequel la coupure à ladite étape (4) est réalisée en utilisant un procédé chimique choisi parmi un traitement acide, un traitement basique ou le chauffage.

5. Procédé selon la revendication 1, dans lequel la coupure à ladite étape (4) est réalisée en utilisant un dispositif de coupure choisi dans le groupe comprenant un générateur d'ondes ultrasoniques, un générateur d'ondes de cisaillement et un accélérateur de particules.

6. Procédé selon la revendication 1, dans lequel le dNMP à ladite étape (5) est quantifié en utilisant un dispositif de mesure choisi dans le groupe comprenant une chromatographie liquide haute performance (CLHP), une chromatographie liquide couplée à une spectrométrie de masse (CL/SM), une chromatographie sur couche mince (CCM) et une chromatographie gazeuse (CG).

7. Procédé d'analyse destiné à classifier ou à diagnostiquer le vieillissement, un cancer, des pathogènes, une contamination par des pathogènes, des tissus et des unités élémentaires dans un échantillon, le procédé comprenant l'étape consistant à mesurer le taux de méthylation dans l'échantillon en utilisant le procédé d'analyse quantitative selon l'une quelconque des revendications 1 à 6, et l'étape consistant à comparer la teneur en méthyl cytosine mesurée avec la teneur en méthyl cytosine appartenant à la base de données concernant le vieillissement, un cancer, des pathogènes, des tissus ou des unités élémentaires.
